# EUROPÄISCHE PATENTSCHRIFT

(11) **EP 1 799 665 B1**
(45) Veröffentlichungstag und Bekanntmachung des Hinweises auf die Patenterteilung: **19.08.2009**
(21) Anmeldenummer: 05790930.1
(22) Anmeldetag: 14.10.2005
(51) Int. Cl.: C07D 295/20, C07C 311/19, C07C 303/40

(54) **VERFAHREN ZUR REINIGUNG VON 3-HYDROXY-AMIDINO-PHENYLALANIN-DERIVATEN DURCH AUSFÄLLUNG UND UMKRISTALLISATION EINES SALZES MIT EINER AROMATISCHEN SULFONSÄURE**
METHOD FOR CLEANING 3-HYDROXYAMIDINOPHENYLALANINE DERIVATIVES BY THE PRECIPITATION AND RECRYSTALLISATION OF A SALT AND AN AROMATIC SULPHONIC ACID
PROCEDE POUR LA PURIFICATION DE DERIVES DE 3-HYDROXY-AMIDINOPHENYLALANINE PAR PRECIPITATION ET RECRISTALLISATION D'UN SEL AU MOYEN D'UN ACIDE SULFONIQUE AROMATIQUE

(30) Priorität: 14.10.2004 EP 04024553
(43) Veröffentlichungstag der Anmeldung: 27.06.2007
(73) Patentinhaber: Wilex AG, 81675 München (DE)
(72) Erfinder: GREIVING, Helmut, 40723 Hilden (DE)
(74) Vertreter: Dey, Michael
(86) Internationale Anmeldenummer: PCT/EP2005/010970
(87) Internationale Veröffentlichungsnummer: WO 2006/042678

(56) Entgegenhaltungen:
- EP-A- 0 074 249
- EP-A- 0 861 826
- WO-A-02/074756
- WO-A-20/04067522

## Beschreibung

Die vorliegende Erfindung betrifft die Herstellung von 3-Hydroxyamidinophenylalanin-Derivaten in hochreiner Form, welche z.B. als Urokinase-Inhibitoren verwendet werden können. Ferner betrifft die vorliegende Erfindung die Verwendung hochreiner 3-Hydroxyamidinophenylalanin-Derivate zur Herstellung von 3-Amidinophenylalanin-Derivaten.

Das Urokinaseplasminogen-Aktivatorsystem (UPA-System) spielt eine zentrale Rolle bei der Metastasierung und darüber hinaus beim Wachstum von Primärtumoren, beispielsweise bei Brust-, Magen-, Darm-, Pankreas-, Eierstockkrebs und anderen soliden Tumoren. Durch eine Inhibierung des UPA-Systems kann sich eine medizinische Wirkung auf zwei Ebenen entfalten: zum einen die Blockierung der Metastasierung sowie zum anderen die Reduzierung des Primärtumorwachstums. 3-Amidinophenylalanin-Derivate stellen eine Klasse von hochwirksamen Urokinase-Inhibitoren dar.

Die Herstellung von 3-Amidinophenylalanin-Derivaten, insbesondere von N-α-(2,4,6-Triisopropyl-phenylsulfonyl)-3-amidino-(L)-phenylalanin-4-ethoxycarbonyl-piperazid (WX-UK1), und deren Verwendung als Urokinase-Inhibitoren ist beispielsweise in CH-A-689611, WO 00/04954 und WO 00/17158 sowie in der Publikation Stürzebecher et al. (Bioorg. Med. Chem. Let 9 (1999), 3147 - 3152) beschrieben. Die dort verwendeten Syntheseverfahren liefern jedoch im Allgemeinen relativ geringe Produktausbeuten, da hydrolysiertes TIPPS-OH als unerwünschtes Nebenprodukt anfällt. Ein Problem besteht darin, dass das erwünschte Reaktionsprodukt nur über aufwändige Chromatographieverfahren von Nebenprodukten getrennt werden kann.

WO 2004/067522 und WO 02/074756 offenbaren Verfahren zur Herstellung und Reinigung von 3-Hydroxyamidinophenylalanin-Derivaten. Es wird eine Aufreinigung über chromatographische Auftrennung beschrieben.

EP 0 861 826 offenbart ein Verfahren zur Reinigung einer Aminosäure,
wobei es sich bei der Aminosäure um eine aliphatische Aminosäure mit einer verzweigten Aminosäurekette handelt.

EP 0 074 249 beschreibt ein Verfahren zur Reinigung von L-Asparagin-p-aminosäuredipeptidamiden, wobei die Dipeptide in Form eines Salzes mit einer aromatischen Sulfonsäure aus einem wässrigen Lösungsmittel selektiv kristallisiert werden.

PCT/EP03/08230 beschreibt Verfahren zur Herstellung von 3-Amidinophenylalanin-Derivaten über eine 3-Hydroxyamidinophenylalanin-Zwischenverbindung. Diese Oxamidin-Derivate stellen hochspezifische und selektive Urokinase-Inhibitoren dar und bieten zudem den Vorteil oraler Bioverfügbarkeit. In dem aus PCT/EP03/08230 bekannten Herstellungsverfahren besteht jedoch das Problem, dass die Oxamidin-Zwischenverbindungen nicht in reiner Form erhalten werden und aufwändige Reinigungsverfahren erforderlich sind, um beispielsweise Amid-Nebenprodukte abzutrennen. Sowohl für die weitere Synthese von 3-Amidinophenylalanin-Derivaten, als auch für eine pharmazeutische Verwendung der Oxamidin-Derivate selbst wäre es jedoch vorteilhaft, ein Verfahren zur Verfügung zu haben, mit dem diese Oxamidin-Derivate in guter Ausbeute und mit hoher Reinheit hergestellt werden können.

Es war daher die Aufgabe der vorliegenden Erfindung, ein solches Verfahren bereitzustellen, um Oxamidin-Derivate in hochreiner Form zu erhalten.

Diese Aufgabe wird gelöst durch ein Verfahren zur Reinigung einer Verbindung der Formel (I), umfassend die Schritte:
(a) Zugeben einer aromatischen Sulfonsäure zu einer Lösung eines ggf. verunreinigten 3-Hydroxyamidinophenylalanin-Derivates um ein Präzipitat zu bilden,
(b) Abtrennen des in Schritt (a) gebildeten Präzipitats, und
(c) Zurückgewinnen des freien 3-Hydroxyamidinophenylalanin-Derivates aus dem Präzipitat,
   wobei die Verbindung die Formel (I) aufweist 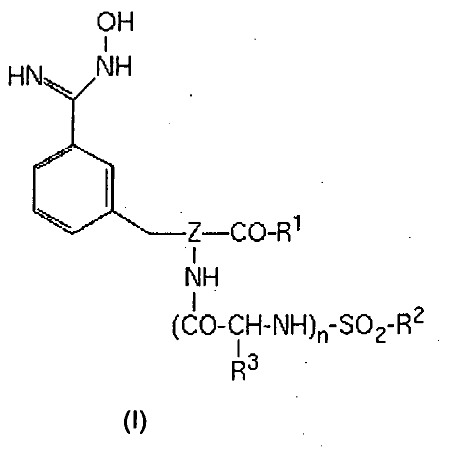 worin R¹ eine Gruppe der Formel 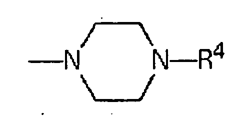 darstellt, in welcher R⁴
   (i) einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogen-substitierten C₁-C₆₋Alkylrest, wie z.B. Ethoxycarbonyl- oder Arylrest, wie z.B. Phenyl, p-Halogenphenyl, Naphtyl,
   (ii) einen gesättigten oder ungesättigten verzweigten oder unverzweigtenC₁-C₆-Alkoxyrest oder
   (iii) einen gegebenenfalls z.B. mit C₁-C₆-Alkyl-, C₁-C₃-Alkoxy, Hydroxyl-, Carboxyl-, Sulfonyl-, Nitro-, Cyano-, Oxo- oder/und Halogen-substituierten Phenoxy- bzw. Benzyloxycarbonylrest bedeutet,
      R² einen gegebenenfalls z.B. mit C₁-C₆-Alkyl-, C₁-C₃-Alkoxy-, Hydroxyl-, Carboxyl, Sulfonyl-, Nitro-, Cyano-, Oxo- oder/und Halogen-substituierten Phenylrest, wie beispielsweise Phenyl, 4-Methyl-phenyl, 2,4,6-Trimethylphenyl, 2,4,6-Triisopropylphenyl, 4-Methoxy-2,3,6-trimethylphenyl darstellt,
      R³ H oder verzweigtes bzw. unverzweigtes C₁-C₄-Alkyl ist,
      n 0 oder 1 bedeutet,
      Z N oder CR⁹ bedeutet, wobei R⁹ H oder verzweigtes oder unverzweigtes C₁-C₄ Alkyl ist.

Im Sinn der vorliegenden Erfindung versteht man unter "aromatische Sulfonsäure" ein aromatisches oder heteroaromatisches mono- oder oligocyclisches Ringsystem, das mit mindestens einer Sulfonsäure-Gruppe und/oder Sulfonat-Gruppe substituiert ist. Das aromatische bzw. heteroaromatische Ringsystem kann außerdem weitere Substituenten tragen, die beispielsweise ausgewählt sein können aus C₁-C₆-Alkyl, C₁-C₃- Alkoxy, Hydroxyl, Carboxyl, Sulfonyl, Nitro, Cyano, Oxo oder/und Halogen. Geeignete aromatische bzw. heteroaromatische Ringsysteme umfassen z.B. Mono- und Bicyclen mit 6 bis 20 Kohlenstoffatomen und 0 bis 4 Heteroatomen, die vorzugsweise ausgewählt sein können aus N, O und S.

Beispiele für geeignete aromatische Sulfonsäuren sind Toluol- und Benzol-Mono- oder/und -Disulfonsäuren sowie Naphthalin-Mono- oder/und -Disulfonsäure-Derivate. Bevorzugt sind Disulfonsäuren mit guter Kristallisationsneigung, beispielsweise Naphtalindisulfonsäuren. Am meisten bevorzugt ist Naphthalin-1,5-disulfonsäure (Armstrong-Säure). Aromatische Disulfonsäuren werden häufig in der Farbstoffindustrie eingesetzt und stellen dort Intermediate und Kupplungsreagenzien verschiedener, insbesondere Naphthalin-basierter Farbstoffe dar.

Überraschenderweise wurde nun gefunden, dass sich aromatische Sulfonsäuren und insbesondere Armstrong-Säure besonders gut zur Fällung von einer Verbindung der Formel (I) bzw. von 3-Hydroxyamidinophenylalanin-Derivaten (Oxamidinen) in Form eines entsprechenden Salzes eignen. So gebildete Salze präzipitieren aus einer Lösung von ggf. verunreinigten 3-Hydroxyamidinophenylalanin-Derivat Rohprodukten. Als Lösungsmittel eignen sich dabei z.B. Ketone, wie Aceton und Pentanon, Ester wie Ethylacetat, polare Ether wie Tetrahydrofuran, Bis-(2-methoxyethyl)-ether (DiGlyme), Dioxan und Methyltertbuthylether, halogenierte Lösungsmittel wie Dichlormethan, aber auch Nitrile und unpolare Alkohole. Die Präzipitate können auf einfache Weise in einem darauffolgenden Schritt (b) von der Lösung abgetrennt werden, beispielsweise durch Filtration.

Das abgetrennte Salz kann anschließend durch im Stand der Technik übliche Reinigungsverfahren weiter gereinigt und getrocknet werden. Auf diese Weise kann erfindungsgemäß die Reinheit der Verbindung der Formel (I) bzw. des 3-Hydroxyamidino-phenylalanin-Derivates noch weiter verbessert werden.

Anschließend wird in Schritt (c) die Verbindung der Formel (I) bzw. das 3-Hydroxyamidinophenylalanin-Derivat

zurückgewonnen. Hierfür kann gemäß der vorliegenden Erfindung das entsprechende Sulfonsäuresalz beispielsweise mit Basen, welche basischer sind als die Hydroxyamidinophenylalanin-Derivate, so z.B. mit Natriumhydrogencarbonat, aber auch mit anorganischen und organischen Basen umgesetzt werden.

Somit ermöglicht die vorliegende Erfindung, auf einfache und elegante Art und Weise verunreinigte Edukte, Zwischenverbindungen und unerwünschte Produkte, vor allem unerwünschte Amidverunreinigungen, von dem Oxamidin abzutrennen.

Das Verfahren der vorliegenden Erfindung ist zur Reinigung von Oxamidin-Verbindungen der Formel (I) 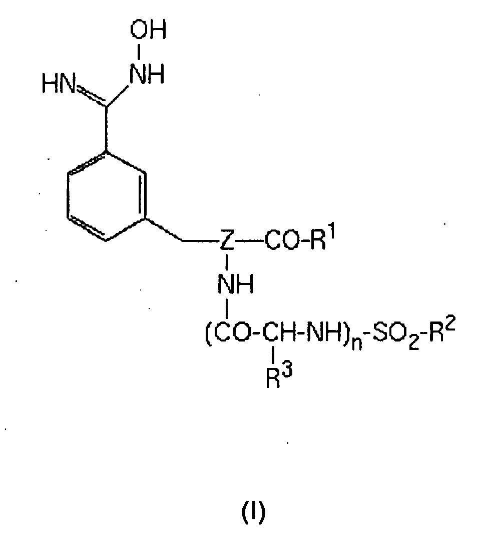 die als Razemate sowie als L- bzw. D-konfigurierte Verbindungen vorliegen können und in denen R¹ eine Gruppe der Formel 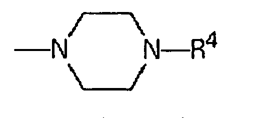 darstellt, in welcher R⁴
(i) einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogen-substituierten C₁-C₆-Alkylrest, wie z.B. Ethoxycarbonyl- oder Arylrest, wie z.B. Phenyl, p-Halogenphenyl, Naphthyl,
(ii) einen gesättigten oder ungesättigten verzweigten oder unverzweigten C₁-C₆-Alkoxyrest oder
(iii) einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogensubstituieren Phenoxy- bzw. Benzyloxycarbonylrest bedeutet,
   R² einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogen-substituierten Phenylrest, wie beispielsweise Phenyl, 4-Methyl-phenyl, 2,4,6-Trimethylphenyl, 2,4,6-Triisopropylphenyl, 4-Methoxy-2,3,6-trimethylphenyl
   darstellt,
   R³ H oder verzweigtes bzw. unverzweigtes C₁-C₄-Alkyl ist und
   n 0 oder 1 bedeutet,
   Z N oder CR⁹ bedeutet, wobei R⁹ H oder verzweigtes oder unverzweigtes C₁-C₄-Alkyl ist, geeignet.

Gemäß einem Aspekt der Erfindung kann es bevorzugt eingesetzt werden, um N-α-(2,4,6-triisopropylphenylsulfonyl)-3-hydroxyamidino-(L)-phenylalanin-4-ethoxy-carbonyl-piperazid in hochreiner Form zu isolieren.

Bei den bisher bekannten Syntheseverfahren zur Herstellung von Oxamidin-Derivaten entsteht die Oxamidin-Verbindung in einem niedrigen Reinheitsgrad und weist einen großen Anteil von bis zu 30 % Amidverunreinigung sowie weitere Verunreinigungen durch Edukte und unbekannte Verbindungen auf. Mit dem Verfahren der vorliegenden Erfindung gelingt es, reines Oxamidin zu erhalten. Bevorzugt entsteht das Oxamidin in mehr als 90 %, weiter bevorzugt mehr als 95 % und am meisten bevorzugt mehr als 99 % Reinheit.

Gemäß einem weiteren Aspekt der vorliegenden Erfindung erfolgt bei dem erfindungsgemäßen Reinigungsverfahren keine Racemisierung, sodass enantiomerenreine Oxamidin-Produkte erhalten werden.

Gemäß einer Ausführungsform der Erfindung kann die erfindungsgemäße Reinigung Teil eines Verfahrens zur Herstellung einer Verbindung der Formel (I) bzw. von 3-Hydroxyamidino-phenylalanin-Derivaten sein. Ein bevorzugtes Herstellungsverfahren umfasst die Schritte:
(i) Umsetzung eines N-geschützten 3-Cyanophenylalanins mit einem Piperazin-Derivat unter Bildung eines N-geschützten 3-Cyanophenylalanin-Piperazids;
(ii) Umsetzung mit einem gegebenenfalls substituierten Phenylsulfonylhalogenid;
(iii) Umwandlung der Cyano-Gruppe in eine Hydroxyamidino-Gruppe
(iv) Reinigung des gebildeten 3-Hydroxyamidinophenylalanin-Derivates nach einem Verfahren gemäß einem der Ansprüche 1 bis 10, wobei die Verbindung die Formel (I) aufweist: 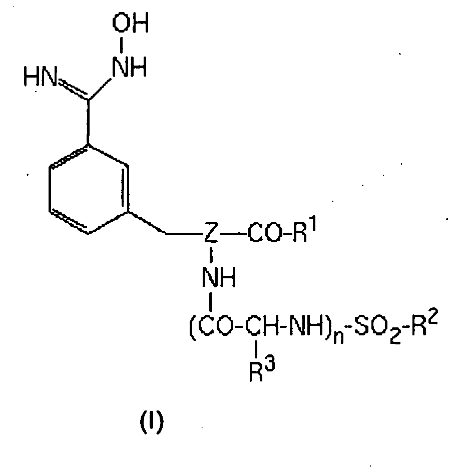
worin R¹ eine Gruppe der Formel 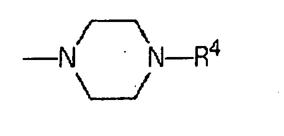 darstellt, in welcher R⁴
(i) einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogensubstitierten C₁-C₆-Alkylrest, wie z.B. Ethoxycarbonyl- oder Arylrest, wie z.B. Phenyl, p-Halogenphenyl, Naphtyl,
(ii) einen gesättigten oder ungesättigten verzweigten oder unverzweigtenC₁-C₆-Alkoxyrest oder
(iii) einen gegebenenfalls z.B. mit C₁-C₆-Alkyl-, C₁-C₃-Alkoxy, Hydroxyl-, Carboxyl-, Sulfonyl-, Nitro-, Cyano-, Oxo- oder/und Halogen-substituierten Phenoxy- bzw. Benzyloxycarbonylrest bedeutet,
   R² einen gegebenenfalls z.B. mit C₁-C₆-Alkyl-, C₁-C₃-Alkoxy-, Hydroxyl-, Carboxyl, Sulfonyl-, Nitro-, Cyano-, Oxo- oder/und Halogen-substituierten Phenylrest, wie beispielsweise
   Phenyl, 4-Methyl-phenyl, 2,4,6-Trimethylphenyl, 2,4,6-Triisopropylphenyl, 4-Methoxy-2,3,6-trimethylphenyl darstellt,
   R³ H oder verzweigtes bzw. unverzweigtes C₁-C₄-Alkyl ist,
   n 0 oder 1 bedeutet,
   Z N oder CR⁹ bedeutet, wobei R⁹ H oder verzweigtes oder unverzweigtes C₁-C₄ Alkyl ist.

Gemäß Schritt (i) des erfindungsgemäßen Verfahrens erfolgt die Umsetzung eines N-geschützten 3-Cyanophenylalanins mit einem Piperazin-Derivat. Die Bezeichnung "Piperazin-Derivat" umfasst im Sinne der vorliegenden Erfindung Piperazin und dessen Derivate, wobei gegebenenfalls bis zu vier Kohlenstoffpositionen des Zyklus und/oder nicht mehr als ein Stickstoffatom des Ringes substituiert sein können. Bevorzugt werden Piperazin-Derivate eingesetzt, die an einem der beiden Stickstoffatome des Ringes einen Substituenten tragen.

Beispiele für geeignete Substituenten umfassen C₁-C₆ Alkylreste, gesättigte oder ungesättigte verzweigte oder unverzweigte C₁-C₆ Alkoxyreste, Phenoxy- bzw. Phenyloxycarbonylreste, und Arylreste wie beispielsweise Phenyl, p-Halogenphenyl und Naphtyl. Diese können ihrerseits jeweils unabhängig gegebenenfalls substituiert sein, zum Beispiel mit C₁-C₆ Alkyl, C₁- C₃ Alkoxy-, Hydroxyl, Carboxyl, Sulfonyl, Nitro, Cyano, Oxo oder/und Halogen.

Gemäß der vorliegenden Erfindung kann eine beliebige Schutzgruppe eingesetzt werden, um das Aminostickstoffatom des in Schritt (i) eingesetzten 3-Cyanophenylalanins zu schützen. Beispiele für geeignete Schutzgruppen für Aminofunktionen sind im Stand der Technik bekannt und umfassen beispielsweise Cbz (Benzyloxycarbonyl), Boc (T-Butyloxycarbonyl), DIMOZ (Dimetoxybenzyloxycarbonyl), Tfac (Trifluoracetyl), CyOC (Cyan-t-butyloxycarbonyl), Phth (Phthaloyl), Bpoc (2-Biphenyl-4-isopropoxycarbonyl), Ddz (3,5-Dimethoxyphenylisopropoxycarbonyl), Fmoc (Fluorenyl-9-methyloxycarbonyl), PALOC (3-(3-Pyridyl)-allyloxycarbonyl), Tos (p-Toluolsulfonyl), NPS (2-Nitrophenylsulfenyl), DNPS (2,4-Dinitrophenylsulfenyl). Gemäß der vorliegenden Erfindung wird besonders bevorzugt die Schutzgruppe Boc eingesetzt.

Bei der Umsetzung in Schritt (i) des erfindungsgemäßen Verfahrens wird ein N-geschütztes 3-Cyanophenylalanin-Piperazid gebildet.

Anschließend kann gemäß einer Ausführungsform der Erfindung die N-Schutzgruppe wieder entfernt werden. Die zur Abspaltung der jeweiligen Schutzgruppe erforderlichen Bedingungen sind einem Fachmann bekannt. Die erfindungsgemäß besonders bevorzugt verwendete Schutzgruppe Boc kann beispielsweise in saurem Medium, z.B. in einem organischen Lösungsmittel wie Dioxan oder Methanol, das mit HCl-Gas oder Trifluoracetat gesättigt ist, besonders bevorzugt mit 4M HCl (g) in Dioxan, abgespalten werden. Alternativ können in dem darauffolgenden Reaktionsschritt (ii) die Bedingungen so gewählt werden, dass in situ eine Abspaltung der Schutzgruppe erfolgt.

In Schritt (ii) wird das in Schritt (i) gebildete 3-Cyanophenylalanin-Piperazid mit einem Phenylsulfonylhalogenid, das gegebenenfalls substituiert sein kann, umgesetzt.

Bevorzugte Halogenide sind dabei Fluorid, Chlorid, Bromid und Jodid. Beispiele für Substituenten an dem Phenylsulfonylhalogenid umfassen C₁-C₆ Alkyl, C₁-C₃ Alkoxy, Hydroxyl, Carboxyl, Sulfonyl, Nitro, Cyano, Oxo und Halogen. Bevorzugt eingesetzte Phenylsulfonylhalogenide sind Phenyl-, 4-Methyl-phenyl-, 2,4,6-Trimethylphenyl-, 4-Methoxy-2,3,6-trimethylphenyl- und insbesondere 2,4,6-Triisopropylphenylsulfonyl (TIPPS)- Halogenide.

In Schritt (iii) erfolgt die Umwandlung der Cyano-Gruppe in die Hydroxyamidinofunktion. Diese Umwandlung kann beispielsweise mittels Hydroxylamin-Hydrochlorid in Gegenwart von Natriumcarbonat oder Triethylamin durchgeführt werden. Geeignete Verfahren für derartige Umsetzungen sind im Stand der Technik bekannt und werden z.B. in WO03/072559 beschrieben.

Das in Schritt (iii) gebildete 3-Hydroxyamidino-phenylalanin-Derivat wird anschließend entsprechend dem oben beschriebenen Reinigungsverfahren mittels einer organischen Sulfonsäure in hochreiner Form erhalten. Es ist dabei nicht erforderlich, das 3-Hydroxyamidino-phenylalanin-Derivat aus Schritt (iii) zunächst zu isolieren.

Auf diese Weise werden 3-Hydroxyamidino-phenylalanin-Derivate unter einem geringen apparativen Aufwand in hoher chemischer Ausbeute und Reinheit erhalten.

Die mit dem erfindungsgemäßen Verfahren in hochreiner Form erhaltenen Oxamidin-Verbindungen können z.B. als oral verfügbare Urokinase-Inhibitoren eingesetzt werden.

Gemäß einem weiteren Aspekt der vorliegenden Erfindung können die Oxamidin-Verbindungen weiter umgesetzt werden zu 3-Amidinophenylalanin-Derivaten. Da gemäß der vorliegenen Erfindung Oxamidin in hochreiner Form vorliegt, ist auch die Ausbeute an Amidino-Endprodukt im weiteren Syntheseverlauf deutlich erhöht.

Die vorliegende Erfindung stellt somit ein Verfahren zur Herstellung von 3-Amidinophenylalanin-Derivaten bereit, umfassend die Schritte:
(i) Herstellung eines 3-Hydroxyamidinophenylalanin-Derivats gemäß einem oben beschriebenen Verfahren, und
(ii) Umwandlung der Hydroxyamidino-Gruppe in eine Amidino-Gruppe.

Die Hydroxyamidinogruppe kann durch Reduktion in das Amidinoderivat überführt werden. Dies erfolgt üblicherweise durch katalytische Hydrierung gemäß Reaktionen die dem Fachmann geläufig sind, bspw. wie in WO 03/076391, WO 03/072559, EP 1 294 742, sowie bei Steinmetzer et al., J. Enzyme Inhibition, 16, 2001, 241-249, Kent et al., J. Peptide Res., 52, 1998, 201-207 und Stüber et al., Peptide Res., 8, 1995, 78-85, angegeben.

Durch das Verfahren der Erfindung kann somit das entsprechende 3-Amidinophenylalanin-Derivat mit gegenüber den bekannten Verfahren des Stands der Technik deutlich verbesserter Ausbeute und in hochreiner Form isoliert werden.

### Figuren

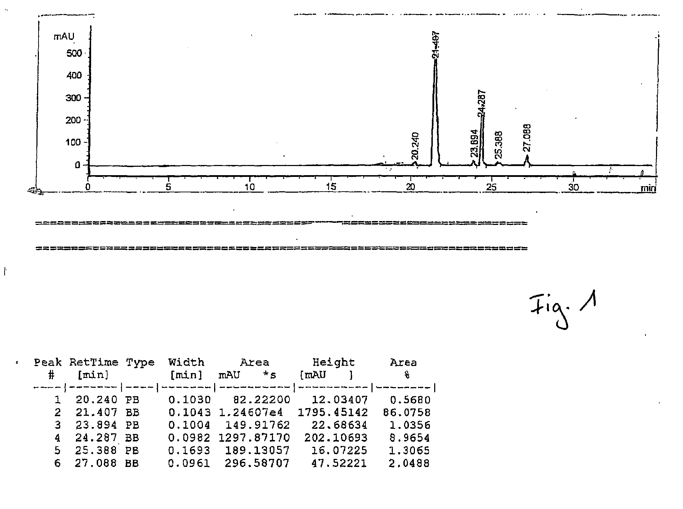

Figur 1 zeigt ein HPLC-Profil des bei einer herkömmlichen Synthese von N-alpha-(2,4,6-triisopropylphenylsulfonyl)-3-hydroxyamidino-(L)-phenylalanin-4-ethoxycarbonylpiperazid erhaltenen Produktgemisches. Das Oxamidin wird bei einer Retentionszeit von ca. 21,4 Minuten detektiert, bei 24,3 Minuten folgt die Amidverunreinigung.

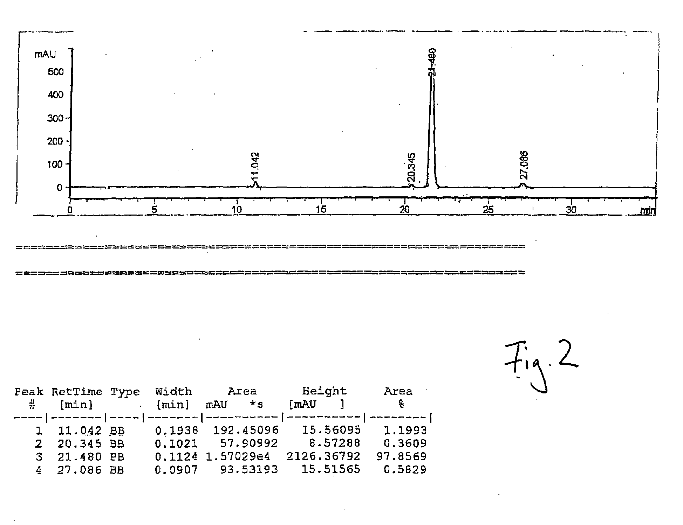

Figur 2 zeigt ein HPLC-Profil des nach dem erfindungsgemäßen Verfahren hergestellten Oxamidins N-alpha-(2,4,6-triisopropylphenylsulfonyl)-3-hydroxyamidino-(L)-phenylalanin-4-ethoxycarbonylpiperazid.

### Beispiel

### Schritt 1: Alkylierung und Decarboxylierung

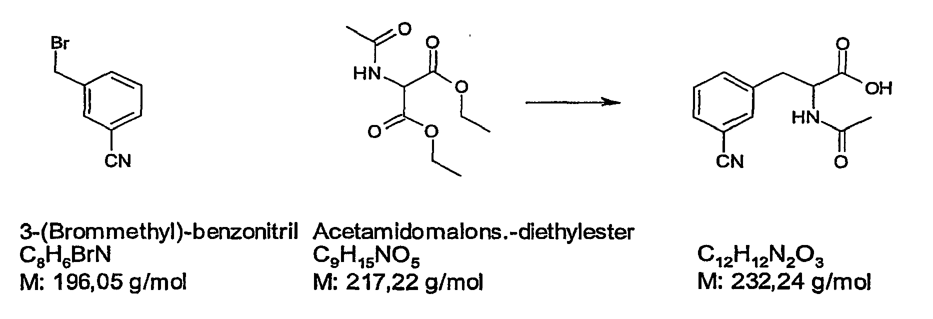

Diethylacetamidomalonat (407 g) wird bei Raumtemperatur in einer Stickstoffatmosphäre zu Ethanol (1 l) zugegeben. Die Suspension wird auf ca. 70 °C erwärmt und es wird eine Lösung von Natriumethoxid (130 g) in Ethanol (900 ml) zugegeben. Die Reaktionsmischung wird für weitere 30 Minuten bei dieser Temperatur gerührt. Anschließend wird eine Suspension von 3-Brommethylbenzonitril (300 g) in Ethanol (1,4 l) zu dem Reaktionsgefäß hinzugefügt. Die Temperatur in dem Reaktionsgefäß wird für weitere 2,5 Stunden bei ca. 70 °C gehalten. Anschließend wird innerhalb 1,5 Stunden verdünnte Natriumhydroxidllösung (2N; 2,2 l) zugetropft. Es wird weitere 30 Minuten bei ca. 70 °C gerührt und anschließend wird die Suspension auf Raumtemperatur gekühlt. Bei Raumtemperatur wird der pH-Wert der Reaktionsmischung langsam auf ca. 7 erniedrigt durch die Zugabe von konzentrierter Salzsäure (innerhalb ca. 1 Stunde). Das organische Lösungsmittel wird durch Destillation unter vermindertem Druck (< 100 mbar; Tₘₐₓ = 60 °C) entfernt. Der verbleibende Rückstand wird in 1 N NaOH (1 l) gelöst. Die wässrige Lösung wird 3 mal mit Ethylacetat (jeweils 350 ml) extrahiert. Anschließend wird auf 10 °C gekühlt. Die wässrige Lösung wird mit konz. HCl angesäuert (pH = 1). Das gewünschte Produkt wird mit Ethylacetat extrahiert (3 Extraktionen mit jeweils 1,2 l Ethylacetat). Die vereinigten organischen Phasen werden unter vermindertem Druck aufkonzentriert. Das gewünschte Produkt fällt als weißer Feststoff aus. Die Kristalle werden bei 5 °C in einem Saugtrichter gesammelt, mit kleinen Mengen an Ethylacetat gewaschen und bei 45 °C in einer Stickstoffatmosphäre getrocknet. Aubeute: 243 g (68 %).

### Schritt 2: Enzymatische Racematspaltung

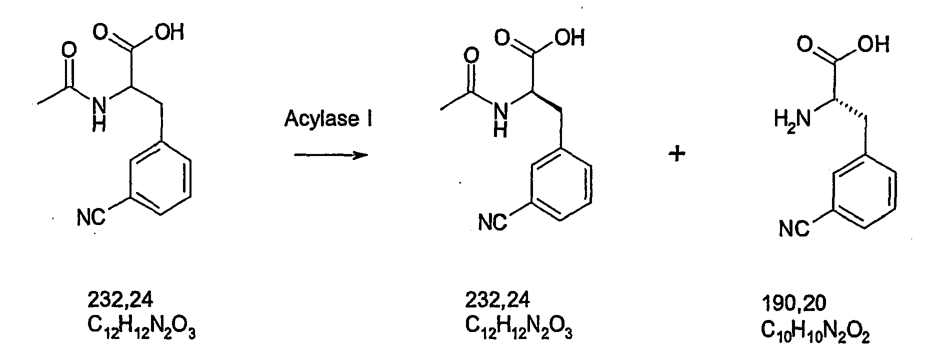

Acetyl-Cyanophenyl-Alanin (940 g) wird in 1 N NaOH (4 l) bei 37 °C gelöst. Der anfängliche pH-Wert von 12,8 wird durch Zugabe von 4N HCl (ungefähr 120 ml) auf 7,2 erniedrigt. Acylase I (37,8 g) wird hinzugefügt. Um den pH-Wert der Reaktionsmischung konstant bei ca. 7,2 zu halten, wird kontinuierlich NaOH (1N) zu der Reaktionsmischung gegeben. Nach 72 Stunden bei 37 °C wird das präzipitierte Produkt durch Filtration bei 20 °C isoliert. Die Kristalle werden mit Wasser gewaschen und bei vermindertem Druck bei ca. 40 °C getrocknet. Wenn das Filtrat unter vermindertem Druck auf ein Drittel des Ursprungsvolumens konzentriert wird, fällt weiteres Produkt aus. Gesamtausbeute: 261 g (34 %).

### Schritt 3: Schützen der NH₂-Funktionalität mit Boc

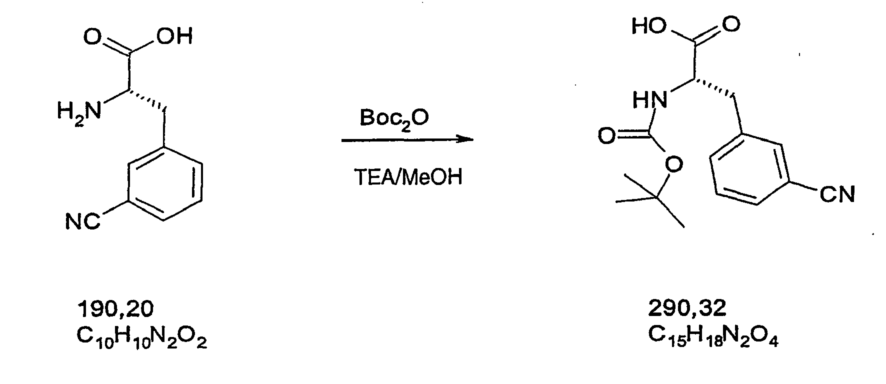

Zu einer Suspension von Cyanophenyl-L-Alanin (425 g) in Methanol (4,5 l) wird Triethylamin (310 ml) hinzugefügt. Bei 25 °C wird Di-tert.-butyldicarbonat (488 g) zugegeben. Die Reaktionsmischung wird über Nacht gerührt. Das organische Lösungsmittel wird bei vermindertem Druck bei 40 ° C entfernt. Das verbleibende orange-farbene Öl wird mit Ethylacetat (3 l) verdünnt. 1N HCl (2,3 l) wird zugegeben und die heterogene Lösung wird für 30 Minuten stark gerührt. Man führt eine Phasentrennung durch. Die organische Phase wird isoliert, mit Wasser extrahiert und mit MgSO₄ getrocknet. Nach Filtration wird die organische Phase bei 40 °C unter vermindertem Druck zur Trockene verdampft. Es werden Kristalle erhalten, wenn Dichlormethan zu dem Rückstand zugegeben wird. Die Kristalle werden durch Filtration gesammelt und unter Stickstoff bei 45 °C getrocknet. Aubeute: 554 g (85 %)

### Schritt 4: Kupplungsreaktion

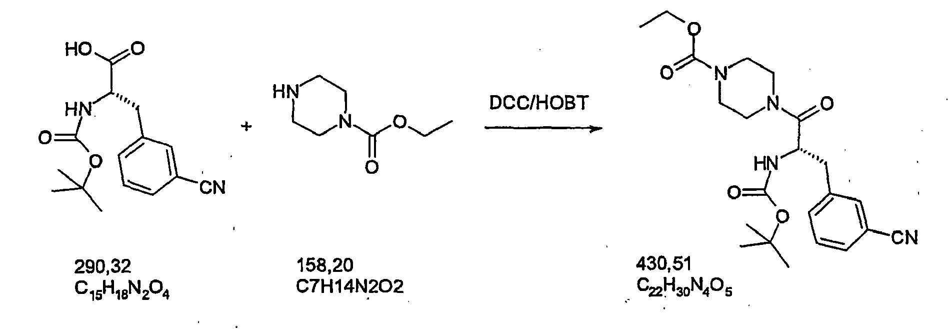

Boc-Cyanophenylalanin (554 g) und HOBT (52 g) werden in Dichlormethan (2,7 l) suspendiert. Eine Lösung von DCC (453 g) in Dichlormethan (1 l) wird zugegeben. Anschließend wird die Suspension auf 10 - 15 °C gekühlt und es wird innerhalb 30 Minuten Ethoxycarbonylpiperazin (347 g) zugetropft, während der obige Temperaturbereich beibehalten wird. Die Reaktionsmischung wird über Nacht gerührt. Der Harnstoff wird abfiltriert und verworfen. Gesättigte NaHCO₃-Lösung (1,8l) wird zu dem Filtrat zugegeben. Die heterogene Mischung wird für 30 Minuten gerührt und anschließend werden die Phasen getrennt. Die organische Phase wird mit Wasser (2 l) extrahiert und anschließend mit MgSO₄ getrocknet. Das MgSO₄ wird abfiltriert und die Lösung wird unter vermindertem Druck bei 30 °C konzentriert. Das verbleibende Öl wird in Ethylacetat (300 ml) gelöst. Die Lösung wird zum Siedepunkt erhitzt. Diisopropylether (750 ml) wird langsam zugegeben, bis die Lösung sich trübt. Die Temperatur wird auf 50 °C erniedrigt und die Mischung wird absitzen gelassen. Innerhalb von 5 Stunden wird die Temperatur anschließend auf 25 °C erniedrigt. Das Produkt wird abfiltriert und mit Diisopropylether gewaschen und anschließend unter Stickstoff bei 40 °C getrocknet. Ausbeute: 660 g (80 %)

### Schritt 5: Entschützen

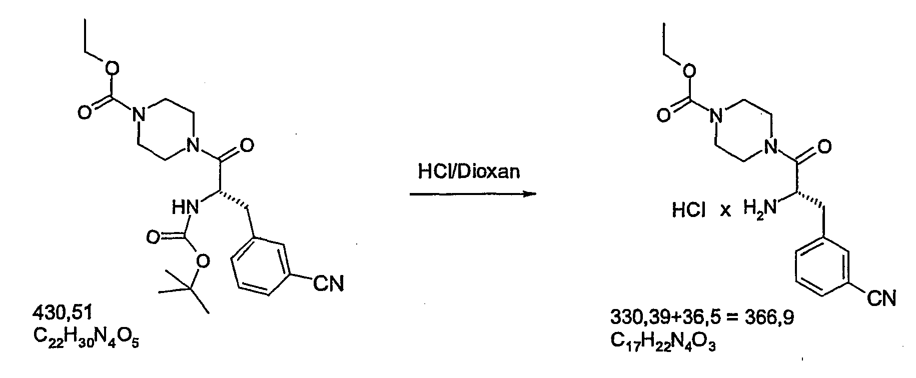

Boc-L-Cyanophenylalanin-Pipamid (442 g) wird in einer Lösung von HCl in Dioxan (4N; 1,2 l) gelöst. Die Temperatur steigt während der Zugabe von 25 °C auf 32 °C. Nach 3 Stunden ist die Reaktion beendet und Dichlormethan (1 l) wird zu der Lösung zugegeben. Das gewünschte Produkt beginnt auszufallen. Die Suspension wird über Nacht gerührt. Ein Überschuss an HCl wird durch Evakuieren der Mischung entfernt. Das Produkt wird durch Filtration isoliert, mit Diisopropylether (0,7 l) gewaschen und im Hochvakuum bei 45 °C getrocknet. Ausbeute: 552 g (98 %)

### Schritt 6: Sulfonamidbildung

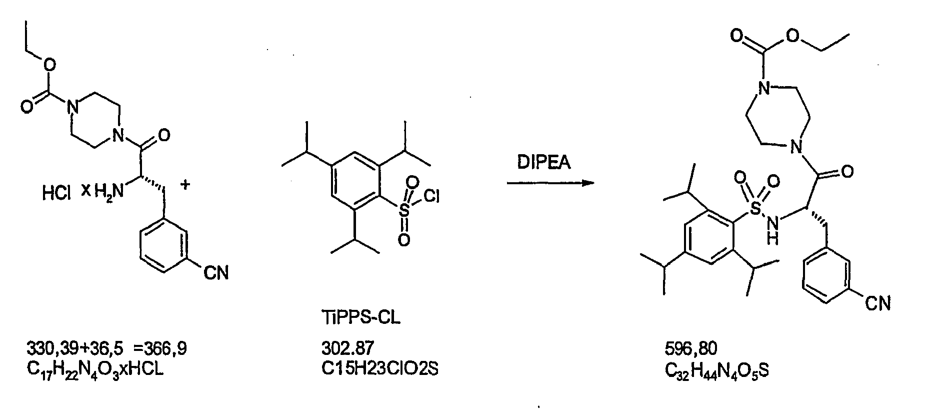

2,4,6-Triisopropylphenylsulfonylchlorid (212 g) wird zu einer Suspension von Cyanophenyl-L-Alanin-Pipamid-HCl (257 g) in Dichlormethan (1,6 l) bei 25 ° C zugegeben. Nach Zugabe von N-Ethyl-diisopropylamin (238 ml) wird eine klare Lösung erhalten. Die Zugabe verläuft exotherm (Temperaturerhöhung von 25 °C auf 35 °C). Wasser (1,1 l) wird zu der Reaktionsmischung hinzugefügt, nachdem sie für 2 Stunden bei Raumtemperatur gerührt wurde. Die Phasen werden getrennt und die organische Phase wird einmal mit gesättigter NaHCO₃-Lösung (1,6 l) und einmal mit Wasser (0,5 l) extrahiert. Die organische Phase wird mit MgSO₄ getrocknet, filtriert und bei vermindertem Druck konzentriert. Es wird ein farbloses Öl erhalten, das beim Stehen an Raumtemperatur langsam auskristallisiert. Ausbeute: 423 g (∼ 100 %)

### Schritt 7: Amidoxim-Bildung; N-alpha-(2,4,6-triisopropylphenylsulfonyl)-3-hydroxyamidino-(L)-phenylalanin-4-ethoxycarbonylpiperazid

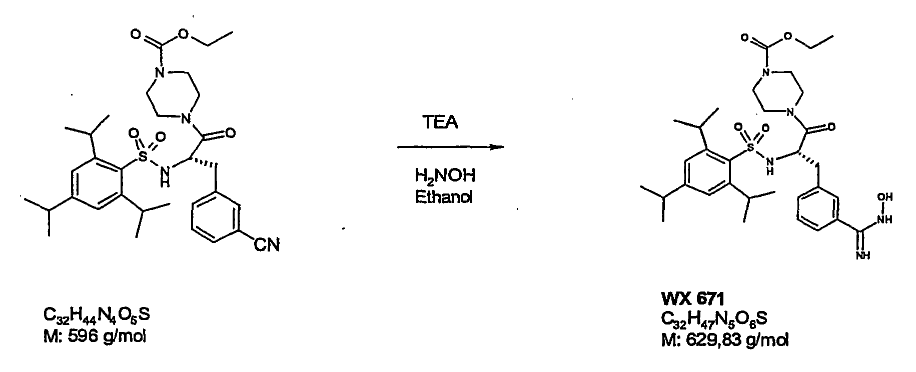

TIPPS-L-Cyanophenylalanin-Pipamid (50 g) wird in Ethanol (350 ml) gelöst. Es werden nacheinander Hydroxylamin-HCl (7,3 g) und Triethylamin (14,5 ml) hinzugefügt. Die Reaktionsmischung wird zum Siedepunkt erwärmt (∼ 75 °C) und für 6 Stunden unter Rückfluss erhitzt. Anschließend wird auf 40 °C gekühlt und das Lösungsmittel wird ausgetauscht. Ethanol wird unter vermindertem Druck entfernt und zu dem Rückstand werden Dichlormethan (300 ml) und Wasser (100 ml) zugegeben. Man führt eine Phasentrennung durch. Die organische Phase wird mit MgSO₄ getrocknet. Nach einer Filtration wird das Lösungsmittel unter vermindertem Druck entfernt.

Es wird ein weißer Feststoff (57 g) erhalten, der in Aceton (200 ml) gelöst wird. Es wird eine Lösung von Armstrong-Säure (15 g) in Aceton (150 ml) zugegeben. Die Mischung wird für 30 Minuten auf den Siedepunkt von Aceton erwärmt. Das Armstrong-Salz des gewünschten Produkts kristallisiert als weißer Feststoff. Die Suspension wird auf Raumtemperatur gekühlt und für 1 Stunde gerührt, bevor anschließend eine Filtration durchgeführt wird. Die Kristalle werden mit Aceton (75 ml) gewaschen, getrocknet und anschließend in Dichlormethan (600 ml) gelöst. Es wird gesättigte NaHCO₃-Lösung (400 ml) zugegeben. Die heterogene Mischung wird für 20 Minuten stark gerührt und anschließend werden die Phasen getrennt. Die organische Phase wird mit Wasser (400 ml) extrahiert, anschließend wird das Dichlormethan durch Destillation entfernt. Ein weißer Feststoff (45 g) wird erhalten. Das Produkt wird aus Ethylacetat/Diisopropylether umkristallisiert, 45 g sind in Ethylacetat (60 ml) gelöst. Diisopropylether (250 ml) wird zugegeben. Die so gebildete Suspension wird für 30 Minuten zum Siedepunkt erhitzt und anschließend langsam auf Raumtemperatur gekühlt. Der amorphe weiße Feststoff wird abfiltriert, mit Diisopropylether gewaschen und bei 45 °C im Vakuum getrocknet. Ausbeute: 34 g (65 %)

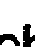

## Patentansprüche

1. Verfahren zur Reinigung einer Verbindung der Formel (I), umfassend die Schritte:
(a) Zugeben einer aromatischen Sulfonsäure zu einer Lösung eines ggf. verunreinigten 3-Hydroxyamidinophenylalanin-Derivates um ein Präzipitat zu bilden,
(b) Abtrennen des in Schritt (a) gebildeten Präzipitats, und
(c) Zurückgewinnen des freien 3-Hydroxyamidinophenylalanin-Derivates aus dem Präzipitat,
wobei die Verbindung die Formel (I) aufweist 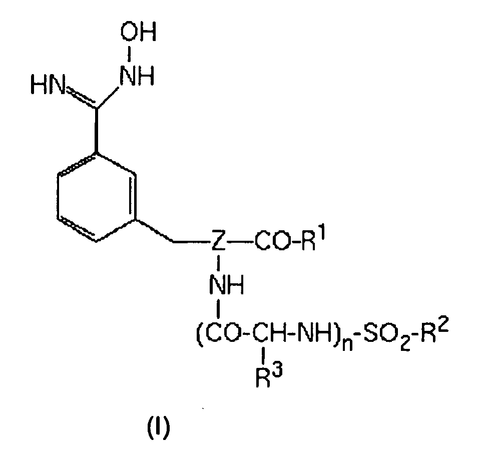 worin R¹ eine Gruppe der Formel 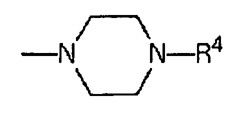 darstellt, in welcher R⁴
(i) einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogensubstitierten C₁-C₆-Alkylrest, wie z.B. Ethoxycarbonyl- oder Arylrest, wie z.B. Phenyl, p-Halogenphenyl, Naphtyl,
(ii) einen gesättigten oder ungesättigten verzweigten oder unverzweigtenC₁-C₆-Alkoxyrest oder
(iii) einen gegebenenfalls z.B. mit C₁-C₆-Alkyl-, C₁-C₃-Alkoxy, Hydroxyl-, Carboxyl-, Sulfonyl-, Nitro-, Cyano-, Oxo- oder/und Halogen-substituierten Phenoxy- bzw. Benzyloxycarbonylrest bedeutet,
R² einen gegebenenfalls z.B. mit C₁-C₆-Alkyl-, C₁-C₃-Alkoxy-, Hydroxyl-, Carboxyl, Sulfonyl-, Nitro-, Cyano-, Oxo- oder/und Halogen-substituierten Phenylrest, wie beispielsweise Phenyl, 4-Methyl-phenyl, 2,4,6-Trimethylphenyl, 2,4,6-Triisopropylphenyl, 4-Methoxy-2,3,6-trimethylphenyl darstellt,
R³ H oder verzweigtes bzw. unverzweigtes C₁-C₄-Alkyl ist,
n 0 oder 1 bedeutet,
Z N oder CR⁹ bedeutet, wobei R⁹ H oder verzweigtes oder unverzweigtes C₁-C₄ Alkyl ist.

2. Verfahren nach Anspruch 1,
worin
die aromatische Sulfonsäure in Schritt (a) Armstrongsäure ist.

3. Verfahren nach einem der vorhergehenden Ansprüche,
worin
die Verbindung der Formel (I) ein N-α-(2,4,6-Triisopropyl-phenylsulfonyl-3-hydroxyamidino-(D,L)-phenylalanin-Derivat, oder das L-Enantiomer davon ist.

4. Verfahren nach einem der vorhergehenden Ansprüche,
worin
die Verbindung der Formel (I) N-α-TIPPS-3-hydroxyamidino-(D,L)-phenylalanin-4-ethoxy-carbonylpiperazid, oder das L-Enantiomer davon ist.

5. Verfahren nach einem der vorhergehenden Ansprüche,
worin
in Schritt (a) eine Lösung der Verbindung der Formel (I) in Aceton eingesetzt wird.

6. Verfahren nach einem der vorhergehenden Ansprüche,
worin
die Abtrennung des Präzipitats in Schritt (b) durch Filtration erfolgt.

7. Verfahren nach einem der vorhergehenden Ansprüche,
worin
die Rückgewinnung in Schritt (c) durch Umsetzung mit NaHCO₃ erfolgt.

8. Verfahren nach einem der vorhergehenden Ansprüche,
worin
die Verbindung der Formel (I) in mehr als 90%-iger Reinheit erhalten wird.

9. Verfahren nach einem der vorhergehenden Ansprüche,
worin
die Verbindung der Formel (I) in mehr als 95%-iger Reinheit erhalten wird.

10. Verfahren nach einem der vorhergehenden Ansprüche,
worin
das Hydroxyamidinophenylalanin-Derivat in mehr als 99%-iger Reinheit erhalten wird.

11. Verfahren zur Herstellung einer Verbindung der Formel (I) umfassend die Schritte:
(i) Umsetzung eines N-geschützten 3-Cyanophenylalanins mit einem Piperazin-Derivat unter Bildung eines N-geschützten 3-Cyanophenylalanin-Piperazids;
(ii) Umsetzung mit einem gegebenenfalls substituierten Phenylsulfonylhalogenid;
(iii) Umwandlung der Cyano-Gruppe in eine Hydroxyamidino-Gruppe
(iv) Reinigung des gebildeten 3-Hydroxyamidinophenylalanin-Derivates nach einem Verfahren gemäß einem der Ansprüche 1 bis 10, wobei die Verbindung die Formel (I) aufweist: 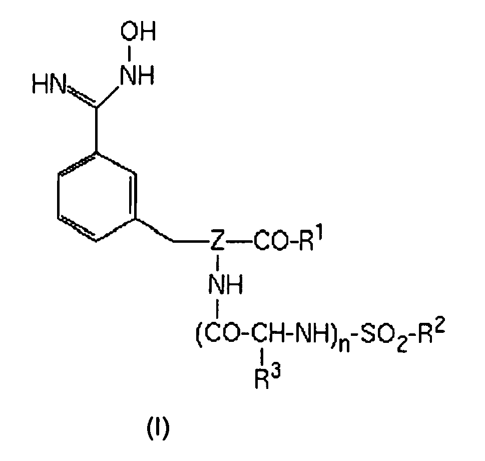 worin R¹ eine Gruppe der Formel 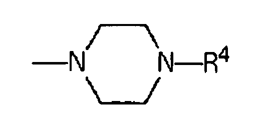 darstellt, in welcher R⁴
(i) einen gegebenenfalls z.B. mit C₁-C₆-Alkyl-, C₁-C₃-Alkoxy-, Hydroxyl-, Carboxyl-, Sulfonyl-, Nitro-, Cyano-, Oxo- oder/und Halogensubstitierten C₁-C₆₋Alkylrest, wie z.B. Ethoxycarbonyl- oder Arylrest, wie z.B. Phenyl, p-Halogenphenyl, Naphtyl,
(ii) einen gesättigten oder ungesättigten verzweigten oder unverzweigtenC₁-C₆-Alkoxyrest oder
(iii) einen gegebenenfalls z.B. mit C₁-C₆-Alkyl-, C₁-C₃-Alkoxy, Hydroxyl-, Carboxyl-, Sulfonyl-, Nitro-, Cyano-, Oxo- oder/und Halogen-substituierten Phenoxy- bzw. Benzyloxycarbonylrest bedeutet,
R² einen gegebenenfalls z.B. mit C₁-C₆-Alkyl-, C₁-C₃-Alkoxy-, Hydroxyl-, Carboxyl, Sulfonyl-, Nitro-, Cyano-, Oxo- oder/und Halogen-substituierten Phenylrest, wie beispielsweise Phenyl, 4-Methyl-phenyl, 2,4,6-Trimethylphenyl, 2,4,6-Triisopropylphenyl, 4-Methoxy-2,3,6-trimethylphenyl darstellt,
R³ H oder verzweigtes bzw. unverzweigtes C₁-C₄-Alkyl ist,
n 0 oder 1 bedeutet,
Z N oder CR⁹ bedeutet, wobei R⁹ H oder verzweigtes oder unverzweigtes C₁-C₄ Alkyl ist.

12. Verfahren nach Anspruch 11,
worin
in Schritt (i) ein mit Boc N-geschütztes 3-Cyanophenylalanin-Derivat eingesetzt wird.

13. Verfahren nach Anspruch 11 oder 12,
worin
das Piperazin-Derivat in Schritt (i) Ethoxycarbonylpiperazin ist.

14. Verfahren nach einem der Ansprüche 11 bis 13,
worin
in Schritt (ii) Umsetzung mit 2,4,6-Triisopropylbenzolsulfonylchlorid erfolgt.

15. Verfahren nach einem der Ansprüche 11 bis 14,
worin
die Cyano-Gruppe in Schritt (iii) mittels Hydroxylamin-Hydrochlorid in Gegenwart von Natriumcarbonat oder Triethylamin in eine Hydroxyamidino-Gruppe umgewandelt wird.

16. Verfahren nach einem der Ansprüche 11 bis 15,
worin
die Verbindung der Formel (I) in mehr als 90%-iger Reinheit entsteht.

17. Verfahren nach einem der Ansprüche 11 bis 16,
worin
die Verbindung der Formel (I) in mehr als 95%-iger Reinheit entsteht.

18. Verfahren nach einem der Ansprüche 6 bis 10,
worin
die Verbindung der Formel (I) in mehr als 99%-iger Reinheit entsteht.

19. Verfahren zur Herstellung von 3-Amidinophenylalanin-Derivaten umfassend die Schritte:
(i) Herstellung einer Verbindung der Formel (I) gemäß einem Verfahren nach einem der Ansprüche 8 bis 18, und
(ii) Umwandlung der Hydroxyamidino-Gruppe in eine Amidino-Gruppe.

20. Verfahren nach Anspruch 19,
worin
die Hydroxyamidino-Gruppe in Schritt (ii) durch Umsetzung mit Acetanhydrid und anschließende katalytische Hydrierung in eine Amidino-Gruppe umgewandelt wird.

## Claims

1. A method for purifying a compound of the formula (I), comprising the steps:
(a) addition of an aromatic sulfonic acid to a solution of an optionally contaminated 3-hydroxyamidinophenylalanine derivative to form a precipitate,
(b) separation of the precipitate formed in step (a), and
(c) recovery of the free 3-hydroxyamidinophenylalanine derivative from the precipitate,
the compound being of the formula (I) 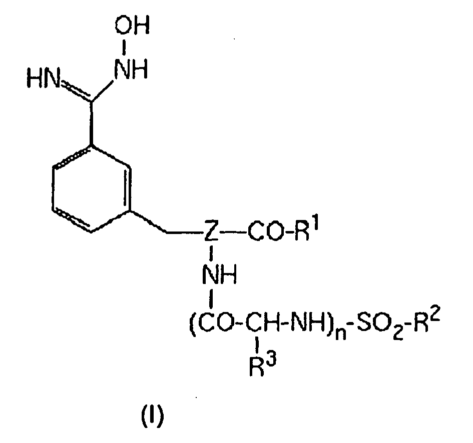 in which R¹ represents a group of the formula 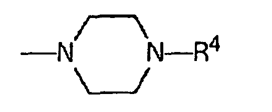 in which R⁴ means
(i) a C₁-C₆ alkyl residue optionally substituted for example with C₁-C₆ alkyl, C₁-C₃ alkoxy, hydroxyl, carboxyl, sulfonyl, nitro, cyano, oxo and/or halogen, such as for example ethoxycarbonyl or aryl residue, such as for example phenyl, p-halophenyl, naphthyl,
(ii) a saturated or unsaturated branched or unbranched C₁-C₆ alkoxy residue or
(iii) a phenoxy- or benzyloxycarbonyl residue optionally substituted for example with C₁-C₆ alkyl, C₁-C₃ alkoxy, hydroxyl, carboxyl, sulfonyl, nitro, cyano, oxo and/or halogen,
R² represents a phenyl residue optionally substituted for example with C₁-C₆ alkyl, C₁-C₃ alkoxy, hydroxyl, carboxyl, sulfonyl, nitro, cyano, oxo and/or halogen, such as for example phenyl, 4-methylphenyl, 2,4,6-trimethylphenyl, 2,4,6-triisopropylphenyl, 4-methoxy-2,3,6-trimethylphenyl,
R³ is H or branched or unbranched C₁-C₄ alkyl,
n means 0 or 1,
Z means N or CR⁹, R⁹ being H or branched or unbranched C₁-C₄ alkyl.

2. A method according to claim 1, in which the aromatic sulfonic acid in step (a) is Armstrong's acid.

3. A method according to either of the preceding claims, in which the compound of the formula (I) is an N-α-(2,4,6-triisopropylphenylsulfonyl-3-hydroxyamidino-(D,L)-phenylalanine derivative, or the L-enantiomer thereof.

4. A method according to any one of the preceding claims, in which the compound of the formula (I) is N-α-TIPPS-3-hydroxyamidino-(D,L)-phenylalanine-4-ethoxycarbonylpiperazide, or the L-enantiomer thereof.

5. A method according to any one of the preceding claims, in which a solution of the compound of the formula (I) in acetone is used in step (a).

6. A method according to any one of the preceding claims, in which separation of the precipitate in step (b) proceeds by filtration.

7. A method according to any one of the preceding claims, in which recovery in step (c) proceeds by reaction with NaHCO₃.

8. A method according to any one of the preceding claims, in which the compound of the formula (I) is obtained in greater than 90% purity.

9. A method according to any one of the preceding claims, in which the compound of the formula (I) is obtained in greater than 95% purity.

10. A method according to any one of the preceding claims, in which the hydroxyamidinophenylalanine derivative is obtained in greater than 99% purity.

11. A method for producing a compound of the formula (I) comprising the steps:
(i) reaction of an N-protected 3-cyanophenylalanine with a piperazine derivative to form an N-protected 3-cyanophenylalanine piperazide;
(ii) reaction with an optionally substituted phenylsulfonyl halide;
(iii) conversion of the cyano group into a hydroxyamidino group
(iv) purification of the 3-hydroxyamidinophenylalanine derivative formed by a method according to any one of claims 1 to 10, the compound being of the formula (I): 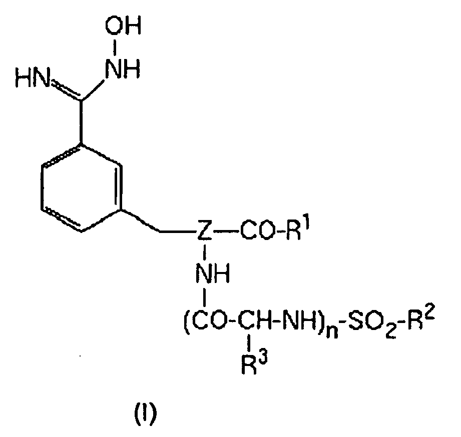 in which R¹ represents a group of the formula 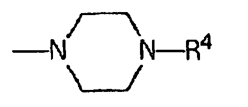 in which R⁴ means
(i) a C₁-C₆ alkyl residue optionally substituted for example with C₁-C₆ alkyl, C₁-C₃ alkoxy, hydroxyl, carboxyl, sulfonyl, nitro, cyano, oxo and/or halogen, such as for example ethoxycarbonyl or aryl residue, such as for example phenyl, p-halophenyl, naphthyl,
(ii) a saturated or unsaturated branched or unbranched C₁-C₆ alkoxy residue or
(iii) a phenoxy- or benzyloxycarbonyl residue optionally substituted for example with C₁-C₆ alkyl, C₁-C₃ alkoxy, hydroxyl, carboxyl, sulfonyl, nitro, cyano, oxo and/or halogen,
R² represents a phenyl residue optionally substituted for example with C₁-C₆ alkyl, C₁-C₃ alkoxy, hydroxyl, carboxyl, sulfonyl, nitro, cyano, oxo and/or halogen, such as for example phenyl, 4-methylphenyl, 2,4,6-trimethylphenyl, 2,4,6-triisopropylphenyl, 4-methoxy-2,3,6-trimethylphenyl,
R³ is H or branched or unbranched C₁-C₄ alkyl,
n means 0 or 1,
Z means N or CR⁹, R⁹ being H or branched or unbranched C₁-C₄ alkyl.

12. A method according to claim 11, in which a 3-cyanophenylalanine derivative N-protected with Boc is used in step (i).

13. A method according to claim 11 or claim 12, in which the piperazine derivative in step (i) is ethoxycarbonylpiperazine.

14. A method according to any one of claims 11 to 13, in which reaction with 2,4,6-triisopropylbenzenesulfonyl chloride proceeds in step (ii).

15. A method according to any one of claims 11 to 14, in which the cyano group is converted in step (iii) into a hydroxyamidino group by means of hydroxylamine hydrochloride in the presence of sodium carbonate or triethylamine.

16. A method according to any one of claims 11 to 15, in which the compound of the formula (I) arises in greater than 90% purity.

17. A method according to any one of claims 11 to 16, in which the compound of the formula (I) arises in greater than 95% purity.

18. A method according to any one of claims 6 to 10, in which the compound of the formula (I) arises in greater than 99% purity.

19. A method for producing 3-amidinophenylalanine derivatives comprising the steps:
(i) production a compound of the formula (I) by a method according to any one of claims 8 to 18, and
(ii) conversion of the hydroxyamidino group into a amidino group.

20. A method according to claim 19, in which the hydroxyamidino group is converted in step (ii) into an amidino group by reaction with acetic anhydride and subsequent catalytic hydrogenation.

## Revendications

1. Procédé pour purifier un composé de formule (I) comprenant les étapes :
(a) ajouter un acide sulfonique aromatique à une solution d'un dérivé de 3-hydroxyamidinophénylalanine éventuellement souillé pour former un précipité,
(b) séparer le précipité formé dans l'étape (a), et
(c) récupérer le dérivé de 3-hydroxyamidinophénylalanine libre à partir du précipité,
où le composé présente la formule (I) 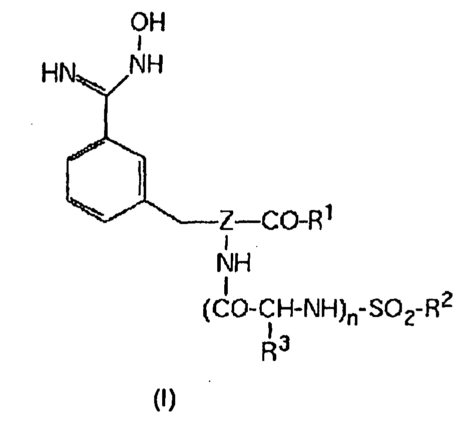 où R¹ représente un groupe de formule 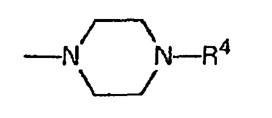 où R⁴ représente
(i) un groupement C₁-C₆-alkyle éventuellement substitué par exemple avec C₁-C₆-alkyle, C₁-C₃-alcoxy, hydroxyle, carboxyle, sulfonyle, nitro, cyano, oxo et/ou halogène, comme par exemple un groupement éthoxycarbonyle ou aryle, comme par exemple phényle, p-halogénophényle, naphtyle,
(ii) un groupement C₁-C₆-alcoxy ramifié ou non ramifié, saturé ou insaturé, ou
(iii) un groupement phénoxy ou benzyloxycarbonyle éventuellement substitué par exemple avec C₁-C₆-alkyle, C₁-C₃-alcoxy, hydroxyle, carboxyle, sulfonyle, nitro, cyano, oxo et/ou halogène,
R² représente un groupement phényle éventuellement substitué par exemple avec C₁-C₆-alkyle, C₁-C₃-alcoxy, hydroxyle, carboxyle, sulfonyle, nitro, cyano, oxo et/ou halogène, comme par exemple phényle, 4-méthyl-phényle, 2,4,6-triméthylphényle, 2,4,6-triisopropylphényle, 4-méthoxy-2,3,6-triméthylphényle,
R³ est H ou C₁-C₄-alkyle ramifié ou non ramifié,
n représente 0 ou 1,
Z représente N ou CR⁹ où R⁹ est H ou C₁-C₄-alkyle ramifié ou non ramifié.

2. Procédé selon la revendication 1 où l'acide sulfonique aromatique dans l'étape (a) est l'acide de Armstrong.

3. Procédé selon l'une des revendications précédentes où le composé de formule (I) est un dérivé de N-α-(2,4,6-triisopropyl-phénylsulfonyl-3-hydroxyamidino-(D,L)-phénylalanine, ou son énantiomère L.

4. Procédé selon l'une des revendications précédentes où le composé de formule (I) est le N-α-TIPPS-3-hydroxy-amidino-(D,L)-phénylalanine-4-éthoxy-carbonylpipérazide, ou son énantiomère L.

5. Procédé selon l'une des revendications précédentes où dans l'étape (a) une solution du composé de formule (I) dans l'acétone est utilisée.

6. Procédé selon l'une des revendications précédentes où la séparation du précipité dans l'étape (b) a lieu par filtration.

7. Procédé selon l'une des revendications précédentes où la récupération dans l'étape (c) a lieu par réaction avec NaHCO₃.

8. Procédé selon l'une des revendications précédentes où le composé de formule (I) est obtenu avec une pureté supérieure à 90 %.

9. Procédé selon l'une des revendications précédentes où le composé de formule (I) est obtenu avec une pureté supérieure à 95 %.

10. Procédé selon l'une des revendications précédentes où le dérivé d'hydroxyamidinophénylalanine est obtenu avec une pureté supérieure à 99 %.

11. Procédé pour produire un composé de formule (I) comprenant les étapes :
(i) réaction d'une 3-cyanophénylalanine N-protégée avec un dérivé de pipérazine avec formation d'un 3-cyanophénylalanine-pipérazide N-protégé ;
(ii) réaction avec un halogénure de phénylsulfonyle éventuellement substitué ;
(iii) conversion du groupe cyano en un groupe hydroxyamidino ;
(iv) purification du dérivé de 3-hydroxyamidinophénylalanine formé selon un procédé selon l'une des revendications 1 à 10, où le composé présente la formule (I) 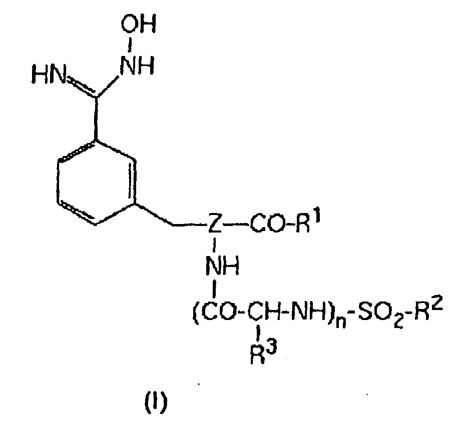 où R¹ représente un groupe de formule 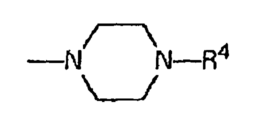 où R⁴ représente
(i) un groupement C₁-C₆-alkyle éventuellement substitué par exemple avec C₁-C₆-alkyle, C₁-C₃-alcoxy, hydroxyle, carboxyle, sulfonyle, nitro, cyano, oxo et/ou halogène, comme par exemple un groupement éthoxycarbonyle ou aryle, comme par exemple phényle, p-halogénophényle, naphtyle,
(ii) un groupement C₁-C₆-alcoxy ramifié ou non ramifié, saturé ou insaturé, ou
(iii) un groupement phénoxy ou benzyloxycarbonyle éventuellement substitué par exemple avec C₁-C₆-alkyle, C₁-C₃-alcoxy, hydroxyle, carboxyle, sulfonyle, nitro, cyano, oxo et/ou halogène,
R² représente un groupement phényle éventuellement substitué par exemple avec C₁-C₆-alkyle, C₁-C₃-alcoxy, hydroxyle, carboxyle, sulfonyle, nitro, cyano, oxo et/ou halogène, comme par exemple phényle, 4-méthyl-phényle, 2,4,6-triméthylphényle, 2,4,6-triisopropylphényle, 4-méthoxy-2,3,6-triméthylphényle,
R³ est H ou C₁-C₄-alkyle ramifié ou non ramifié,
n représente 0 ou 1,
Z représente N ou CR⁹ où R⁹ est H ou C₁-C₄-alkyle ramifié ou non ramifié.

12. Procédé selon la revendication 11 où, dans l'étape (i), un dérivé de 3-cyanophénylalanine N-protégé avec Boc est utilisé.

13. Procédé selon la revendication 11 ou 12 où le dérivé de pipérazine dans l'étpe (i) est l'éthoxycarbonylpipérazine.

14. Procédé selon l'une des revendications 11 à 13 où, dans l'étape (ii), une réaction avec le chlorure de 2,4,6-triisopropylbenzènesulfonyle a lieu.

15. Procédé selon l'une des revendications 11 à 14 où le groupe cyano dans l'étape (iii) est converti avec le chlorhydrate d'hydroxylamine en un groupe hydroxyamidino en présence de carbonate de sodium ou de triéthylamine.

16. Procédé selon l'une des revendications 11 à 15 où le composé de formule (I) apparaît avec une pureté supérieure à 90 %.

17. Procédé selon l'une des revendications 11 à 16 où le composé de formule (I) apparaît avec une pureté supérieure à 95 %.

18. Procédé selon l'une des revendications 6 à 10 où le composé de formule (I) apparaît avec une pureté supérieure à 99 %.

19. Procédé pour produire des dérivés de 3-amidinophénylalanine comprenant les étapes :
(i) production d'un composé de formule (I) selon un procédé selon l'une des revendications 8 à 18, et
(ii) conversion du groupe hydroxyamidino en un groupe amidino.

20. Procédé selon la revendication 19 où le groupe hydroxyamidino dans l'étape (ii) est converti en un groupe amidino par réaction avec l'anhydride acétique puis hydrogénation catalytique.
